# EUROPEAN PATENT SPECIFICATION

(11) **EP 3 370 273 B1**
(45) Date of publication and mention of the grant of the patent: **13.05.2020**
(21) Application number: 16874714.5
(22) Date of filing: 25.11.2016
(51) Int. Cl.: H01L 51/54

(54) **ORGANIC ELECTROPHOSPHORESCENCE DEVICES**
ORGANISCHE ELEKTROLUMINESZVORRICHTUNG
DISPOSITIF À ÉLECTROPHOSPHORESCENCE ORGANIQUE

(30) Priority: 18.12.2015 CN 201510952274
(43) Date of publication of application: 05.09.2018
(73) Proprietor: Kunshan Go-Visionox Opto-Electronics Co., Ltd., KunShan City, Jiangsu 215300 (CN); Tsinghua University, Beijing 100084 (CN)
(72) Inventor: DUAN, Lian, Beijing 100085 (CN); ZHANG, Dongdong, Beijing 100085 (CN); LIU, Song, Beijing 100085 (CN); ZHAO, Fei, Beijing 100085 (CN)
(74) Representative: Hanna Moore + Curley
(86) International application number: PCT/CN2016/107232
(87) International publication number: WO 2017/101657

(56) References cited:
- CN-A- 101 978 528
- US-A1- 2008 315 753
- US-A1- 2015 069 352
- YOUNG-SEO PARK ET AL: "Exciplex-Forming Co-host for Organic Light-Emitting Diodes with Ultimate Efficiency", ADVANCED FUNCTIONAL MATERIALS, WILEY - V C H VERLAG GMBH & CO. KGAA, DE, vol. 23, no. 39, 18 October 2013 (2013-10-18), pages 4914-4920, XP001586852, ISSN: 1616-301X, DOI: 10.1002/ADFM.201300547 [retrieved on 2013-05-06]

## Description

### Technical Field

The present invention belongs to the field of organic electroluminescence devices, and specifically relates to a phosphorescent organic electroluminescence device.

### Background Art

Organic electroluminescence devices have attracted extensive attention due to their features of thinness, large area, full curing and flexibility, and their potential in solid-state lighting sources and liquid crystal backlights has become a focus of research.

As early as in 1950s, Bernanose. A et al. began the research on organic electroluminescence devices (OLEDs). Anthracene single crystal wafer was adopted as the original research materials. Because of large thickness of the single crystal wafer, the driving voltage required is very high. Until 1987, C. W. Tang and Vanslyke from Eastman Kodak Company proposed an organic small molecule electroluminescence device having the structure ITO/Diamine/Alq₃/Mg:Ag. It was reported that when this device worked at a working voltage of 10 V, the luminance reached 1000 cd/m2 and the external quantum efficiency reached 1.0%. The research on electroluminescence has drawn wide attention from scientists, and it is possible for OLEDs to be used in display. Since then, the research on OLEDs and industrialization of OLEDs has been started. An organic luminescent material system includes a fluorescent system and a phosphorescent luminescent system, in which the fluorescent system utilizes singlet state exciton energy alone, and the phosphorescent system can utilize both singlet and triplet state exciton energy.

In a phosphorescent device in which a conventional host exists, energy is transferred from the host at triplet state to a phosphorescent guest at triplet state by short-range Dexter energy transfer, resulting in a higher doping concentration (10 wt%-30 wt%) of the phosphorescent material; although the high doping concentration may reduce the distance between the host and guest and promote complete transfer of energy, the excessive high doping concentration may cause the decrease of device efficiency, also, as the phosphorescent material is made of a noble metal, the high phosphorescent dye doping concentration may increase the cost.

Patent specification US 2015/069352 A1 discloses an organic electroluminescent device with two main luminescent layers.

The technical publication entitled "Exciplex-Forming Co-host for Organic Light-Emitting Diodes with Ultimate Efficiency" (Young-Seo Park et al., Adv. Funct. Mater., 2013, 23, 4914-4920) discloses an organic electroluminescent device with two main luminescent layers.

US 2008/0315753 A1 discloses phosphorescent OLEDs comprising exciton blocking layers between the hole transporting layer and the light emitting layer.

### Technical Problems

The technical problem to be solved by the present invention is that: in a phosphorescent device in which a conventional host exists, energy is transferred from the host at triplet state to a phosphorescent guest at triplet state by short-range Dexter energy transfer, resulting in a higher doping concentration (10 wt%-30 wt%) of the phosphorescent material; the excessive high doping concentration may cause the decrease of device efficiency; also, as the phosphorescent material is made of a noble metal, the high phosphorescent dye doping concentration may increase the cost.

### Technical Solutions

In order to solve the above technical problem, the present invention provides a novel phosphorescent organic electroluminescence device. The phosphorescent organic electroluminescence device includes a hole transport material layer and an electron transport material layer, an exciplex is formed on the interface of the two layers, so that energy is transferred from triplet state of the host material to singlet state of the host by reverse intersystem crossing and then to triplet state of the phosphorescent guest by long-range Förster energy transfer, thereby reducing the doping concentration of a phosphorescent dye.

The phosphorescent organic electroluminescence device provided by the present invention includes a hole transport layer, a luminescent layer and an electron transport layer, which are successively laminated; the luminescent layer has a double-layer structure comprising a hole transport material layer and an electron transport material layer; the hole transport material layer is arranged between the hole transport layer and the electron transport material layer; the electron transport material layer is arranged between the hole transport material layer and the electron transport layer; and an exciplex is formed on the interface of contact between the hole transport material layer and the electron transport material layer;
the hole transport material layer consists of a host material, the host material being a material having hole transport capability;
the electron transport material layer consists of a host material and a phosphorescent material doped in the host material, the host material being a material having electron transport capability;
wherein, the first triplet state energy level of the material having hole transport capability is higher than the first singlet state energy level of the exciplex, with an energy gap between the material having electron transport capability and the exciplex being more than or equal to 0.2eV; and the absolute value of HOMO energy level of the material having hole transport capability is less than or equal to 5.3eV;
the first triplet state energy level of the material having electron transport capability is higher than the first singlet state energy level of the exciplex, with an energy gap between the material having electron transport capability and the exciplex being more than 0.2 eV; and the absolute value of LUMO energy level of the material having electron transport capability is more than 2.0 eV; the difference in LUMO energy level between the material having hole transport capability and the material having electron transport capability is more than 0.3 eV, and the difference in HOMO energy level between the material having hole transport capability and the material having electron transport capability is more than 0.2 eV; and
the first singlet state energy level of the exciplex is higher than the first triplet state energy level of the phosphorescent material.

Preferably, the difference in LUMO energy level between the material having hole transport capability and the material having electron transport capability is more than or equal to 0.4 eV. Wherein, the phosphorescent dye in the luminescent layer account for 1 wt% to 10 wt%, preferably 3 wt%.

Preferably, the thickness ratio of the hole transport material layer and the electron transport material layer is 1:1 to 1:5, preferably 1:3.

Preferably, the material having electron transport capability is one or more of compounds of the formulae:

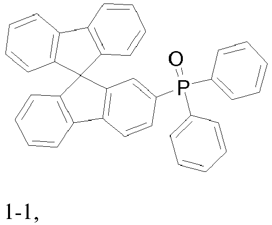

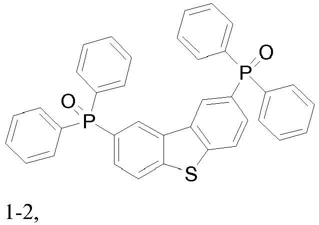

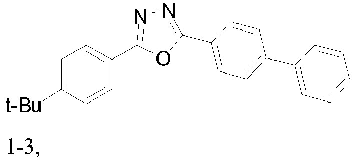

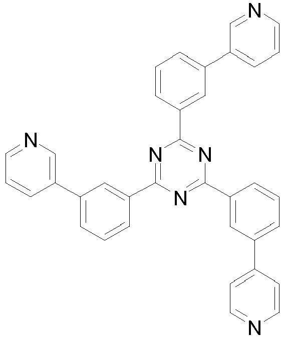

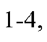

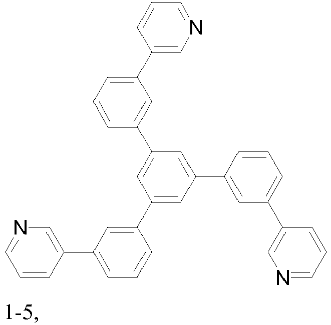

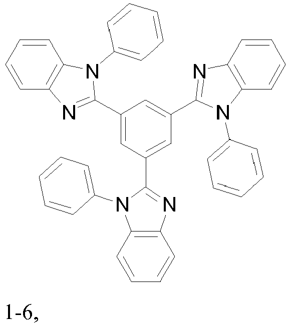

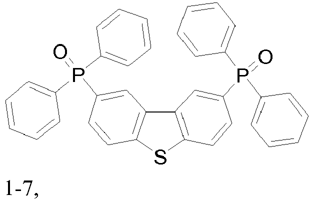

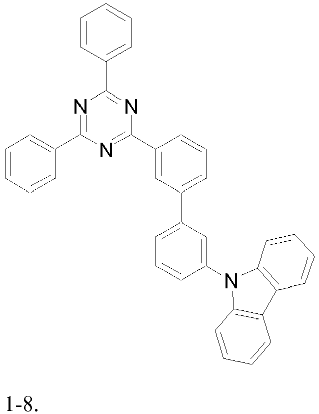

Preferably, the material having hole transport capability is one or more of compounds of the following formulae:

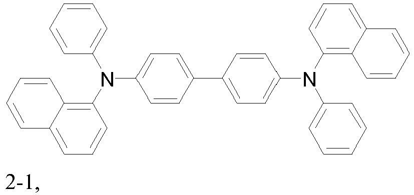

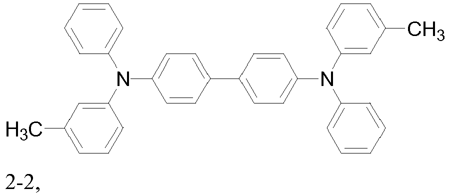

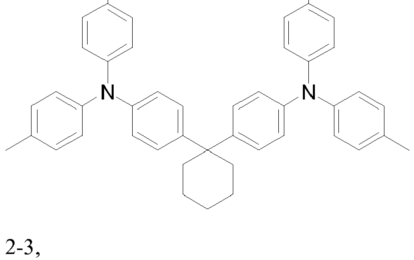

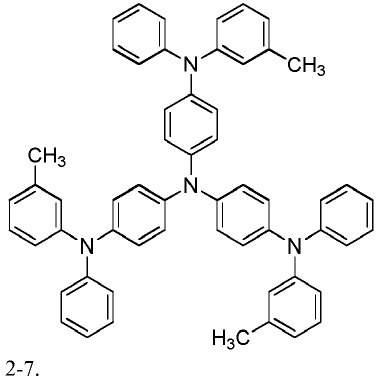

The phosphorescent organic electroluminescence device of the present invention includes an anode, a hole transport layer, the hole transport material layer, the electron transport material layer, an electron transport layer and a cathode, which are successively arranged on a substrate. The anode and the hole transport layer has a hole injection layer arranged therebetween.

### Advantageous Effects

The present invention has the following advantages:
In the phosphorescent organic electroluminescence device of the present invention, the luminescent layer is a double-layer structure and the exciplex is formed on the interface of the hole transport material layer and the electron transport material layer, the exciplex is a TADF exciplex having a thermally activated delayed fluorescence effect, and the triplet state energy of the TADF exciplex is transferred to the singlet state energy by reverse intersystem crossing and then transferred to the triplet state energy of the dopant dye; so that the triplet state energy of the host material and dopant dye in the device can be fully utilized, thereby increasing the device efficiency; and the energy transfer process and luminescent process of thermally activated delayed fluorescence occur in different materials (named as thermally activated sensitization process), so that the problem of significant roll-off under high luminance conditions can be effectively solved, thereby further improving the stability of the device.

In the phosphorescent organic electroluminescence device of the present invention, the luminescent layer utilizes the exciplex to reduce the doping concentration of the phosphorescent dye and also maintain long lifetime and high efficiency.

### Brief Description of the Drawings

Fig. 1 schematically shows a structure of an organic electroluminescence device in accordance to the invention.
Fig. 2 schematically shows energy transfer of a luminescent layer of an organic electroluminescence device not in accordance to the invention.

### Preferred Embodiments of the Invention

The present invention will be further described below with reference to the accompanying drawings and specific embodiments, so that those skilled in the art can better understand and implement the present invention, however, the embodiments are not intended to limit the present invention.

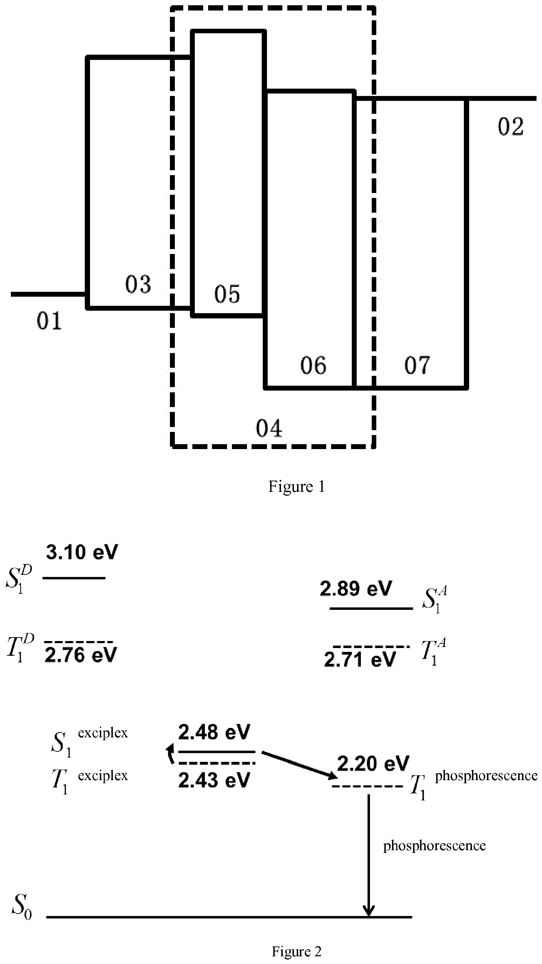

As shown in Fig. 1, the organic electroluminescence device of the invention includes successively depositing on the substrate an anode 01, a hole transport layer 03, a luminescent layer 04, an electron transport layer 07 and a cathode 02 in a way of laminating one by one, wherein the luminescent layer includes a hole transport material layer 05 and an electron transport material layer 06.

The luminescent layer is a double-layer structure formed by the hole transport material layer 05 and the electron transport material layer 06; the hole transport material layer 05 is arranged between the hole transport layer and the electron transport material layer; the electron transport material layer 06 is arranged between a donor layer and the hole transport layer 07; and an exciplex is formed on the interface of contact between the hole transport material layer 05 and the electron transport material layer 06;
the hole transport material layer 05 includes a host material, the host material being a material having hole transport capability;
and the electron transport material layer includes a host material and a phosphorescent dye doped in the host material, the host material being a material having electron transport capability.
In the luminescent layer, the exciplex which is formed between the hole transport material layer 05 and the electron transport material layer 06 meets the following conditions:
T₁^{A}₋S₁ >0.2eV
T₁^{D}-S₁≥0.2eV
|LUMO_{A}|>2.0eV
|HOMO_{D}|≤5.3eV

In the above expressions, T₁^{A} represents the first triplet state energy level of the acceptor (the material having electron transport capability), T₁^{D} represents the first triplet state energy level of the donor (the material having hole transport capability), and S₁ represents the first singlet state energy level of the exciplex, LUMO_{A} represents the LUMO energy level of the acceptor, and HOMO_{D} represents the HOMO energy level of the donor.

In another word, the first triplet state energy level of the material having electron transport capability is higher than the singlet state energy level of the exciplex, with an energy gap between the material having electron transport capability and the exciplex being more than or equal to 0.2eV; and the absolute value of HOMO energy level of the material having hole transport capability is less than or equal to 5.3eV;
the first triplet state energy level of the material having electron transport capability is higher than the first singlet state energy level of the exciplex, with an energy gap between the material having electron transport capability and the exciplex being more than 0.2eV; and the absolute value of LUMO energy level of the material having electron transport capability is more than 2.0eV;
also, the difference in LUMO energy level between the material having hole transport capability and the material having electron transport capability is more than 0.3 eV, preferably more than 0.4 eV;
the difference in HOMO energy level between the material having hole transport capability and the material having electron transport capability is more than 0.2 eV; and the first singlet state energy level of the exciplex is higher than the first triplet state energy level of the phosphorescent dye.

When the exciplex meets the above conditions, it is a thermally activated delayed delayed fluorescence exciplex (TADF exciplex) and has a thermally activated delayed fluorescence effect. The luminescent layer of the present invention has a double-layer structure in which the TADF exciplex is formed on the interface between the hole transport material layer 05 and the electron transport material layer 06, and the triplet state energy of the TADF exciplex is transferred to the singlet state and then transferred to the dopant dye, so that the triplet state energy of the host material and dopant dye in the device can be fully utilized, thereby increasing the device efficiency; and the energy transfer process and luminescence process of thermally activated delayed fluorescence occur in different materials (named as thermally activated sensitization process), so that the problem of significant roll-off under high luminance conditions can be effectively solved, thereby further improving the stability of the device.

As shown in Fig. 2, in the case of using TCTA as donor and mDTPPC as acceptor, the working principle of the luminescent layer will be described below:
The exciplex is generated on the contact interface between the hole transport material layer and the electron transport material layer, so that the excited electrons pass from the first singlet state of the exciplex to the first singlet state of the exciplex by reverse intersystem crossing and then to the first triplet state of the phosphorescent dopant material, emitting a phosphorescent light.

The material having electron transport capability is a compound of the following formulae:

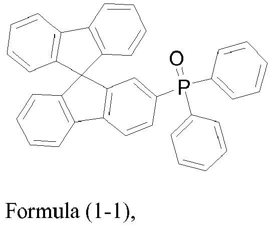

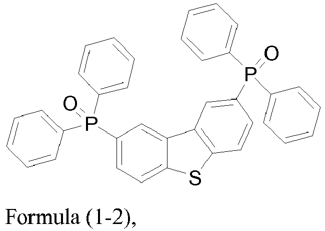

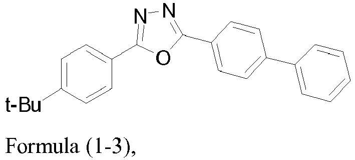

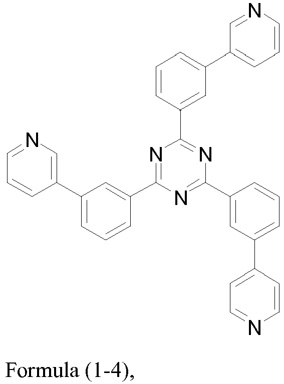

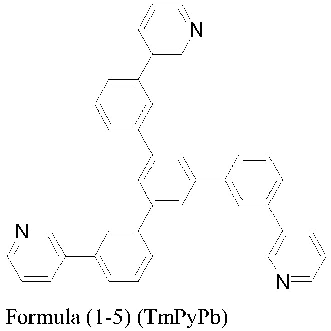

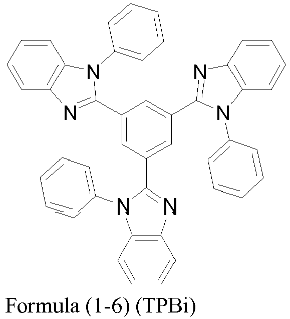

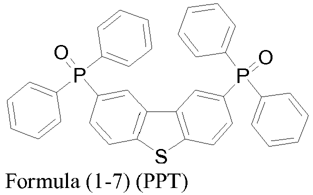

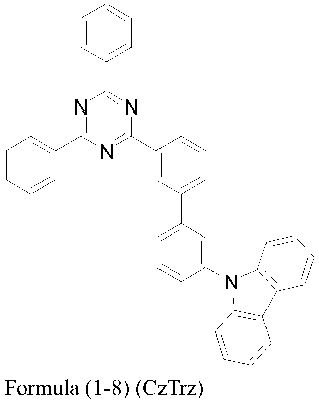

The material having hole transport capability is a compound of the following formulae, where compounds (2-4) - (2-6) do not form part of the invention:

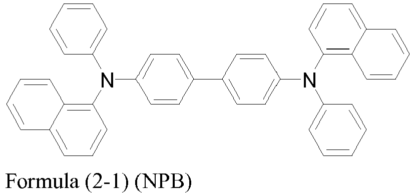

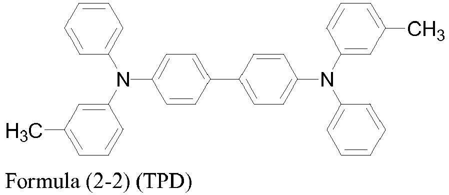

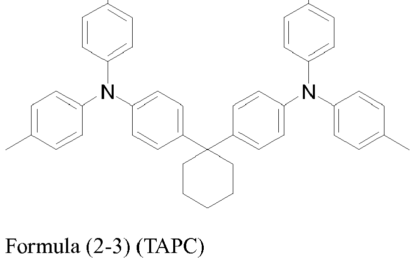

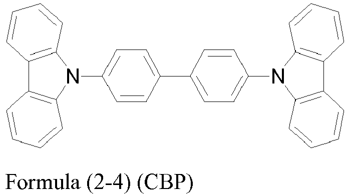

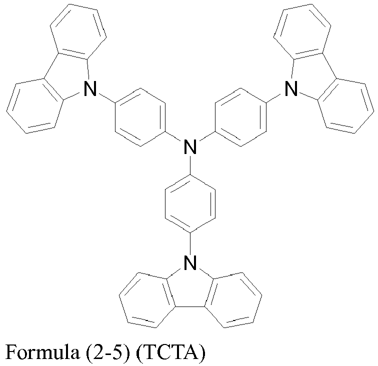

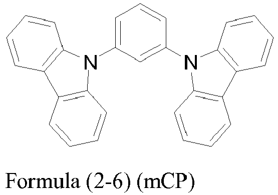

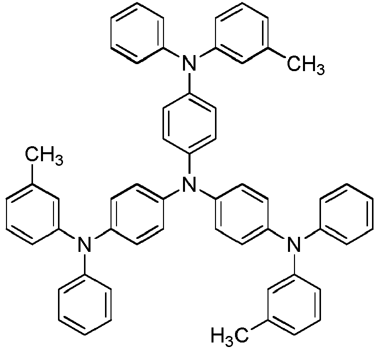

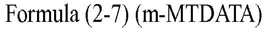

As an example of the organic luminescent display device of the present invention, the anode 01 is made of an inorganic material or organic conductive polymer. The inorganic material is generally a metal oxide such as indium tin oxide (ITO), zinc oxide (ZnO) and indium zinc oxide (IZO), or a metal with higher work functions such as gold, copper and silver, preferably ITO. The organic conductive polymer is preferably one of polythiophene/ polyvinyl sodium benzenesulfonate (hereinafter abbreviated as PEDOT/PSS) and polyaniline (hereinafter abbreviated as PANI).

The cathode 02 is generally made of a metal having lower work functions such as lithium, magnesium, calcium, strontium, aluminum and indium, an alloy thereof with copper, gold or silver, or an electrode layer alternately formed by a metal and a metal fluoride. The cathode 02 in the present invention is preferably a laminate of LiF layer and Al layer (LiF layer on the outer side). The material of the hole transport layer may be selected from aromatic amines and dendrimer-like low molecular materials, preferably NPB.

The material of the electron transport layer may utilize an organic metal complex (such as Alq₃, Gaq₃, BAlq or Ga(Saph-q)) or other materials commonly used in the electron transport layer, such as aromatic fused ring compounds (such as pentacene, germanium) or phenanthrenes (such as Bphen, BCP).

The organic electroluminescence device of the present invention also includes a hole injection layer between the anode 01 and the hole transport layer, and the material of the hole injection layer may be, for example, 4,4',4"-tri(3-methylphenylaniline)triphenylamine-doped F4TCNQ, or copper phthalocyanine (CuPc), or a metal oxide such as molybdenum oxide and yttrium oxide.

The thicknesses of each of the above layers may be conventional for these layers in the art.

The present invention also provides a process for manufacturing the organic electroluminescence device, including successively depositing on the substrate an anode 01, a hole transport layer 03, a luminescent layer 04, an electron transport layer 07 and a cathode 02 in a way of laminating one by one, and then packaging.

The substrate may be a glass or flexible substrate, and the flexible substrate may be made of polyesters or polyimides, or be a thin metal sheet. The laminating and packaging may be completed by any suitable method known to those skilled in the art.

A phosphorescent dye of the luminescent layer in the following examples of the present invention is as follows:
A red phosphorescent dye may be selected from the following compounds:

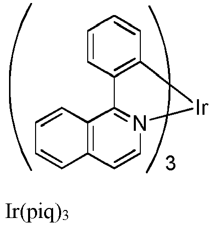

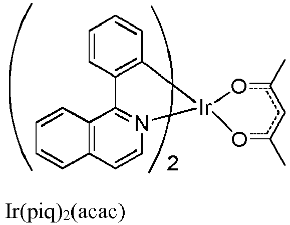

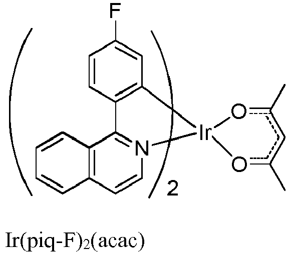

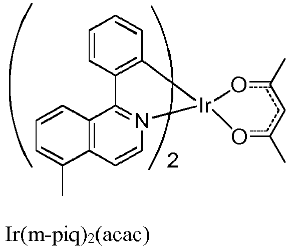

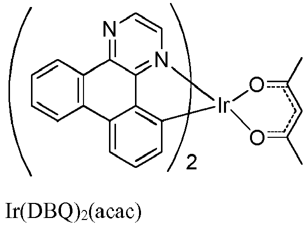

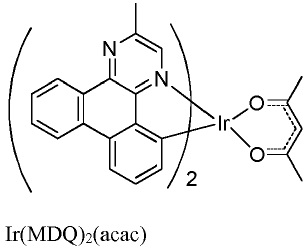

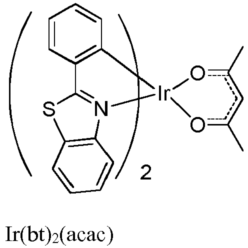

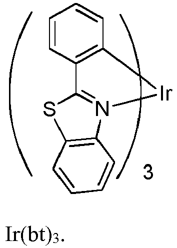

A green phosphorescent dye may be selected from the following compounds:

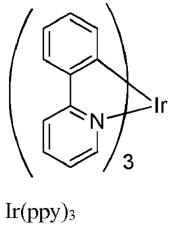

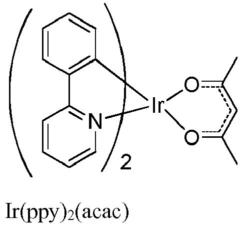

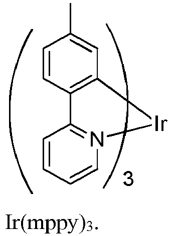

A yellow phosphorescent dye may be selected from the following compounds:

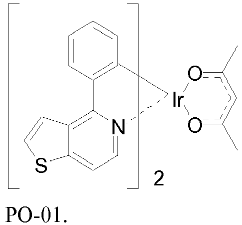

A blue phosphorescent dye used herein may be selected from the following compounds:

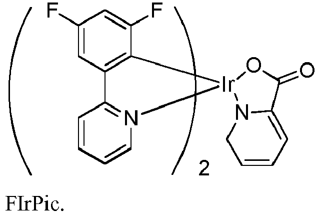

The present invention is further illustrated in connection with the following examples.

### Example 1

In this example, not forming part of the invention, luminescent devices in which a hole transport material layer 05 and an electron transport material layer 06 in a luminescent layer have different thicknesses are manufactured, and these devices have a structure shown as Fig. 1. In the luminescent layer, the hole transport material layer 05 consists of a host material compound (2-5) TCTA, and the electron transport material layer 06 consists of a host material compound (1-8) CzTrz and a phosphorescent dye PO-01. The difference in LUMO energy level between the host compound (2-5) TCTA and the acceptor host compound (1-8) CzTrz is more than 0.3 eV, and the difference in HOMO energy is more than 0.2 eV; and the first singlet state energy level of the exciplex is higher than the first triplet state energy level of the phosphorescent dye. The phosphorescent dye PO-01 accounts for 3 wt% of the luminescent layer.

The device of this example has the following structure.

ITO (150 nm)/NPB (40 nm)/hole transport material layer TCTA (10 nm)/electron transport material layer compound (1-8) CzTrz + phosphorescent dye PO-01 (10-40 nm)/Bphen (20 nm)/LiF (0.5nm)/Al (150 nm)

In this example and the description below, the unit of doping concentration of the phosphorescent dye is wt%.

The organic electroluminescence device was manufactured as follows:
first, a glass substrate was cleaned with a detergent and deionized water and placed under an infrared lamp for drying, and a layer of anode material was sputtered on the glass substrate with a film thickness of 150 nm;
then, the glass substrate having the anode 01 was placed in a vacuum chamber under a pressure of 1×10⁻⁴ Pa, and NPB was vapor deposited on the anode film as a hole transport layer 03 under such conditions, the film forming rate was 0.1 nm/s and the vapor deposited film thickness was 40 nm; a luminescent layer was vapor deposited on the hole transport layer 03 by dual-source co-evaporation, in which a hole transport material layer 05 TCTA (10 nm) was vapor deposited and then an electron transport material layer 06 CzTrz and a phosphorescent dye PO-01 were vapor deposited at the given mass percentages under a film thickness monitor while the film forming rate was controlled and the vapor deposited film thickness was controlled to be 30 nm;
on the luminescent layer, a layer of Bphen material was further vapor deposited as an electron transport layer 07 under such conditions that the vapor deposition rate was 0.1 nm/s and the total thickness of vapor deposited film was 20 nm; and
finally, a LiF layer and an Al layer were successively vapor deposited as a cathode layer of the device by vapor deposition on the luminescent layer, in which the vapor deposition rate of the LiF layer was 0.01-0.02 nm/s and the thickness was 0.5 nm, and the vapor deposition rate of the Al layer was 1.0 nm/s and the thickness was 150 nm.

### Comparative example 1

An organic electroluminescence device was manufactured by the same method as Example 1, having the following structure:
ITO (150 nm)/NPB (40 nm)/CBP + 3 wt% PO-01(40 nm)/Bphen (20 nm)/LiF (0.5 nm)/Al (150 nm)

In another word, the luminescent layer consists of a host material CBP and a phosphorescent dye PO-01, in which the phosphorescent dye PO-01 accounts for 3 wt% of the luminescent layer. In this example and the description below, the unit of doping concentration of the phosphorescent dye is wt%.

### Comparative example 2

An organic electroluminescence device was manufactured by the same method as Example 1, having the following structure:
ITO (150 nm)/NPB (40 nm)/exciplex (TCTA and CzTrz, at the mass ratio of 1:1) + 3 wt% PO-01 (40 nm)/Alq₃ (20 nm)/LiF (0.5 nm)/Al (150 nm)

In another word, the luminescent layer of this comparative example is a single layer consisting of an exciplex as host material and a phosphorescent dye PO-01 doped in the host material, in which the exciplex consists of a material TCTA having hole transport capability and a material CzTrz having electron transport capability, at the mass ratio of 1:1. The phosphorescent dye PO-01 accounts for 3 wt% of the luminescent layer.

The properties of the organic electroluminescence devices of Example 1 and Comparative example 1 are shown in Table 1 below.

**Table 1**

| Device | Luminescent layer composition | Luminous efficiency (cd/A) | Luminanc e (cd/m²) | External quantum efficienc y (%) | Lifetime T₉₇ (hrs) |
|---|---|---|---|---|---|
| Example 1 | TCTA(10)/ CzTrz: 3 wt% PO-01(10 nm) | 58 | 1000 | 19 | 462 |
| | TCTA(10)/ CzTrz: 3 wt% PO-01(20 nm) | 61 | 1000 | 21 | 471 |
| | TCTA(10)/ CzTrz: 3 wt% PO-01(30 nm) | 64 | 1000 | 25 | 480 |
| | TCTA(10)/ CzTrz: 3 wt% PO-01 (40 nm) | 59 | 1000 | 20 | 465 |
| Comparative example 1 | CBP: 3 wt% PO-01(40 nm) | 55 | 1000 | 18 | 437 |
| Comparative example 2 | TCTA: CzTrz: 3 wt% PO-01 (40 nm) | 61 | 1000 | 23 | 450 |

It can be seen from Table 1 that the luminous efficiencies of the devices of both Example 1 and Comparative example 2, using exciplex as host, are higher than that of Comparative example 1 using the conventional host material CBP, and the lifetime of the device using exciplex as host is longer than that of Comparative example 1. The lifetime of Example 1 is longer than that of Comparative example 2, because in the exciplex system of Comparative example 2, the excited state of the donor or acceptor alone is also formed such that the donor or acceptor is easily decomposed, making the device unstable. In addition, Example 1 adopts a double doping system which is easier for control than a triple doping system adopted by Comparative example 2 in terms of process and is suitable for mass production applications.

### Example 2

The device of this example (not forming part of the invention) has the following structure.

ITO (150 nm)/NPB (40 nm)/TCTA (10 nm)/CzTrz+1-10 wt% phosphorescent dye PO-01(30 nm)/Bphen (20 nm)/LiF (0.5 nm)/Al (150 nm)

The phosphorescent dye PO-01 accounts for 1-10 wt% of the luminescent layer.

In this example, different doping concentrations of phosphorescent dye were applied in the luminescent layer for experiments, and the results were shown in Table 2.

**Table 2**

| Luminescent layer | Luminous efficiency (cd/A) | Luminance (cd/m²) | External quantum efficiency (%) | Lifetime T97 (hrs) |
|---|---|---|---|---|
| TCTA(10)/ CzTrz: 1 wt% PO-01(30 nm) | 58 | 1000 | 14.1 | 435 |
| TCTA(10)/ CzTrz: 2 wt% PO-01(30 nm) | 61 | 1000 | 14.7 | 462 |
| TCTA(10)/ CzTrz: 3 wt% PO-01(30 nm) | 64 | 1000 | 16.1 | 480 |
| TCTA(10)/ CzTrz: 5 wt% PO-01(30 nm) | 57 | 1000 | 14.6 | 471 |
| TCTA(10)/ CzTrz: 10 wt% PO-01(30 nm) | 54 | 1000 | 12.9 | 443 |

It can be seen from Table 2 that when the doping concentration of the phosphorescent dye in the luminescent layer is 3 wt%, both the external quantum efficiency and the lifetime of the OLED device are optimal.

### Example 3

In order to evaluate the influence of the host material on the performance of the organic electroluminescence device of the present invention, an organic electroluminescence device was manufactured by the same method as Example 1, having the following structure:
ITO (150 nm)/NPB (40 nm)/hole transport material layer (material having hole transport capability) (10 nm)/electron transport material layer (material having electron transport capability + 3 wt% phosphorescent dye) (30 nm)/Bphen (20nm)/LiF (0.5nm)/Al (150 nm)

The performance of the organic electroluminescence device is shown in Table 3 below (OLED3 not forming part of the invention):

**Table 3**

| Device | Hole transport material layer | Electron transport material layer | Luminous efficiency (cd/A) | Luminance (cd/m²) | External quantum efficiency (%) | Lifetime T97 (hrs) |
|---|---|---|---|---|---|---|
| OLED1 | compound (2-7) | compound (1-3) + phosphorescent dye Ir(piq)₃ | 23 | 1000 | 18 | 390 |
| OLED2 | compound (2-7) | compound (1-7) + phosphorescent dye Ir(DBQ)₂(acac) | 20 | 1000 | 16 | 348 |
| OLED3. | compound (2-5) | compound (1-1) + phosphorescent dye Ir(ppy)₃ | 67 | 1000 | 17 | 390 |
| OLED4 | compound (2-1) | compound (1-3) + phosphorescent dye Ir(mppy)₃ | 63 | 1000 | 16 | 420 |
| OLED5. | compound (2-2) | compound (1-5) + phosphorescent dye FIrPic | 38 | 1000 | 14 | 65 |

It can be seen from Table 3 that the devices of OLED1 to OLED5 in which the exciplex is formed as host on the interface between the hole transport material layer 05 and the electron transport material layer 06 have excellent electrical properties and increased lifetime, indicating that the triplet state energy of the TADF exciplex that is formed on the interface between the hole transport material layer 05 and the electron transport material layer 06 is transferred to the singlet state and then transferred to the triplet state of the dopant material, so that the triplet state energy of both the host material and the dopant material are fully utilized, thereby improving the device efficiency. The scope of the present invention is defined by the claims.

## Claims

1. A phosphorescent organic electroluminescence device, including a hole transport layer, a luminescent layer and an electron transport layer, which are successively laminated, wherein the luminescent layer has a double-layer structure comprising a hole transport material layer and an electron transport material layer, defining an interface between the hole transport material layer and the electron transport material layer, and an exciplex is formed on the interface of the hole transport material layer and the electron transport material layer, the exciplex is a TADF exciplex having a thermally activated delayed fluorescence effect; and the hole transport material layer consists of a host material having hole transport capability, the electron transport material layer consists of a host material and a phosphorescent dye doped in the host material, the host material having electron transport capability; wherein the hole transport material layer is arranged between the hole transport layer and the electron transport material layer, the electron transport material layer is arranged between the hole transport material layer and the electron transport layer wherein, the first triplet state energy level of the material having electron transport capability is higher than the singlet state energy level of the exciplex, with an energy gap between the first triplet of the material having electron transport capability and the first singlet state energy level of the exciplex being more than or equal to 0.2 eV; and the absolute value of HOMO energy level of the material having hole transport capability is less than or equal to 5.3 eV;
the first triplet state energy level of the material having electron transport capability is higher than the first singlet state energy level of the exciplex, with an energy gap between the material having electron transport capability and the exciplex being more than 0.2 eV; and the absolute value of LUMO energy level of the material having electron transport capability is more than 2.0 eV; the difference in LUMO energy level between the material having hole transport capability and the material having electron transport capability is more than 0.3 eV, and the difference in HOMO energy level between the material having hole transport capability and the material having electron transport capability is more than 0.2 eV;
wherein, the material having electron transport capability is one or more of compounds of the structural formulae (1-1) to (1-8): 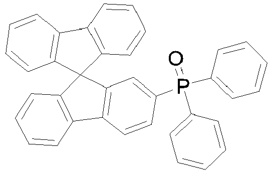 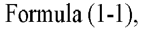 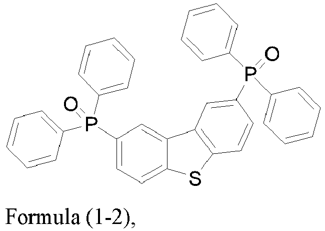 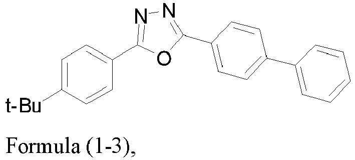 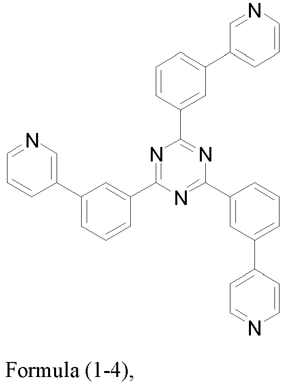 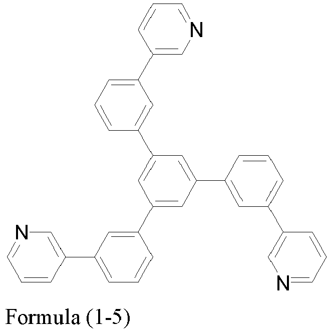 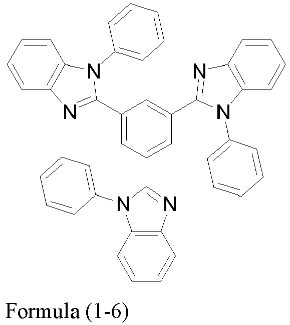 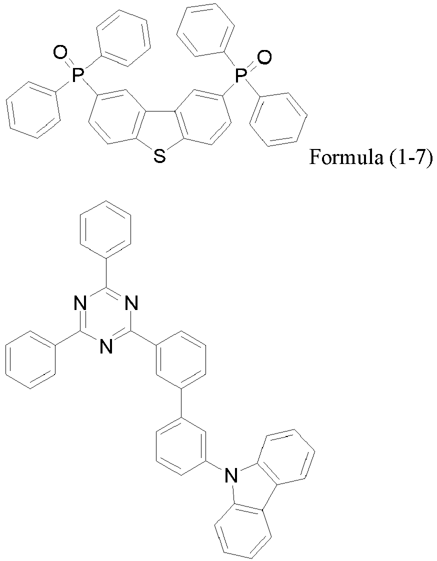 Formula (1-8); wherein, the material having hole transport capability is one or more of compounds of the structural formulae (2-1) to (2-7): 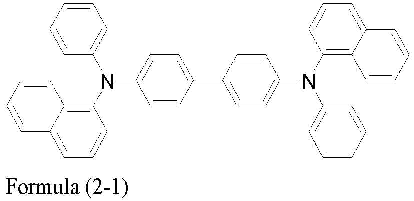 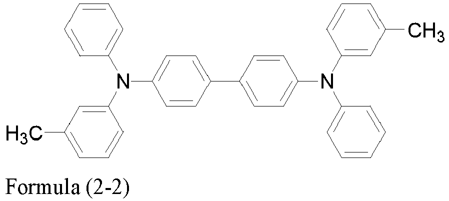 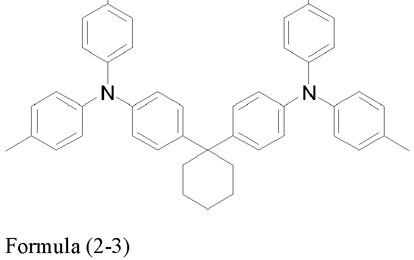 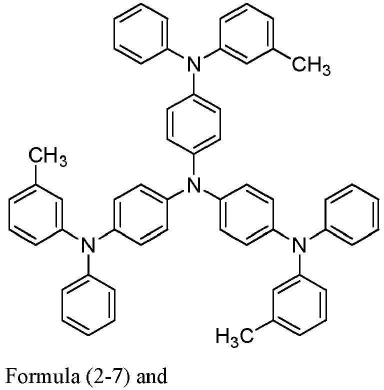 the first singlet state energy level of the exciplex is higher than the first triplet state energy level of the phosphorescent dye.

2. The phosphorescent organic electroluminescence device according to claim 1, wherein the difference in LUMO energy level between the material having hole transport capability and the material having electron transport capability is more than or equal to 0.4 eV.

3. The phosphorescent organic electroluminescence device according to claim 1, wherein the phosphorescent dye accounts for 1-10 wt% of the luminescent layer.

4. The phosphorescent organic electroluminescence device according to claim 1, wherein the phosphorescent dye accounts for 3 wt% of the luminescent layer.

5. The phosphorescent organic electroluminescence device according to claim 1, wherein the thickness ratio of the hole transport material layer to the electron transport material layer is 1:1 to 1:5.

6. The phosphorescent organic electroluminescence device according to claim 1, wherein the thickness ratio of the hole transport material layer to the electron transport material layer is 1:3.

7. The phosphorescent organic electroluminescence device according to claim 1, wherein the phosphorescent organic electroluminescence device includes an anode, a hole transport layer, the hole transport material layer, the electron transport material layer, an electron transport layer and a cathode, which are successively laminated on a substrate.

8. The phosphorescent organic electroluminescence device according to claim 7, wherein the anode and the hole transport layer has a hole injection layer arranged therebetween.

## Patentansprüche

1. Phosphoreszierende organische Elektrolumineszenzvorrichtung, die eine Lochleistungsschicht, eine lumineszierende Schicht und eine Elektronenleistungsschicht beinhaltet, die nacheinander laminiert sind, wobei die lumineszierende Schicht eine Doppelschichtstruktur aufweist, die eine Lochleistungsmaterialschicht und eine Elektronenleistungsmaterialschicht umfasst, die eine Grenzfläche zwischen der Lochleistungsmaterialschicht und der Elektronenleistungsmaterialschicht definiert, und wobei ein Exciplex auf der Grenzfläche der Lochleistungsmaterialschicht und der Elektronenleistungsmaterialschicht ausgebildet ist, wobei der Exciplex ein TADF-Exciplex ist, der einen thermisch aktivierten verzögerten Fluoreszenzeffekt aufweist; und
wobei die Lochleistungsmaterialschicht aus einem Wirtsmaterial besteht, das eine Lochleistungsfähigkeit aufweist, die Elektronenleistungsmaterialschicht aus einem Wirtsmaterial und einem in das Wirtsmaterial dotierten phosphoreszierenden Farbstoff besteht, wobei das Wirtsmaterial eine Elektronenleistungsfähigkeit aufweist;
wobei die Lochleistungsmaterialschicht zwischen der Lochleistungsschicht und der Elektronenleistungsmaterialschicht angeordnet ist, die Elektronenleistungsmaterialschicht zwischen der Lochleistungsmaterialschicht und der Elektronenleistungsschicht angeordnet ist, wobei das erste Triplettzustandsenergieniveau des Materials, das die Elektronenleistungsfähigkeit aufweist, höher als das Singulettzustandsenergieniveau des Exciplex ist, wobei eine Energielücke zwischen dem ersten Triplett des Materials, das die Elektronenleistungsfähigkeit aufweist, und dem ersten Singulettzustandsenergieniveau des Exciplex wenigstens 0,2 eV ist; und
der absolute Wert des HOMO-Energieniveaus des Materials mit Lochleistungsfähigkeit aufweist, höchstens 5,3 eV ist;
wobei das erste Triplettzustandsenergieniveau des Materials, das die Elektronenleistungsfähigkeit aufweist, höher als das erste Singulettzustandsenergieniveau des Exciplex ist, wobei eine Energielücke zwischen dem Material, das die Elektronenleistungsfähigkeit aufweist, und dem Exciplex mehr als 0,2 eV ist; und
der absolute Wert eines LUMO-Energieniveaus des Materials, das die Elektronenleistungsfähigkeit aufweist, mehr als 2,0 eV ist;
wobei die Differenz in dem LUMO-Energieniveau zwischen dem Material, das die Lochleistungsfähigkeit aufweist, und dem Material, das die Elektronenleistungsfähigkeit aufweist, mehr als 0,3 eV ist, und die Differenz in dem HOMO-Energieniveau zwischen dem Material, das die Lochleistungsfähigkeit aufweist, und dem Material, das die Elektronenleistungsfähigkeit aufweist, größer als 0,2 eV ist;
wobei das Material mit Elektronenleistungsfähigkeit aufweist, eine oder mehrere Verbindungen der Strukturformeln (1-1) bis (1-8) ist: 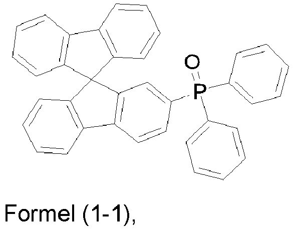 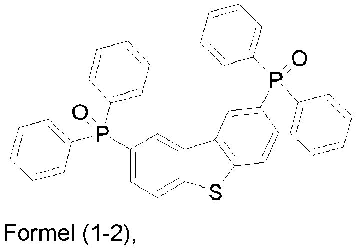 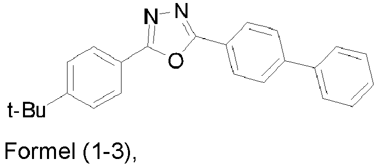 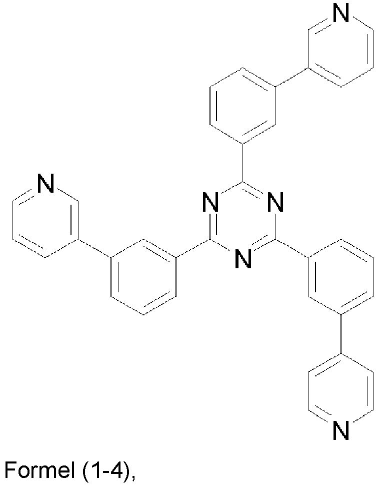 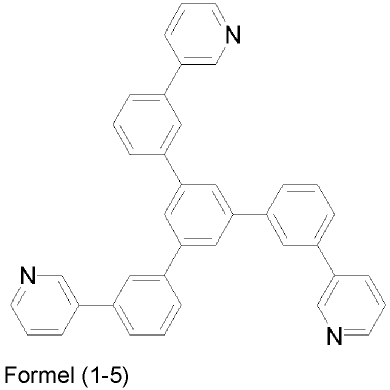 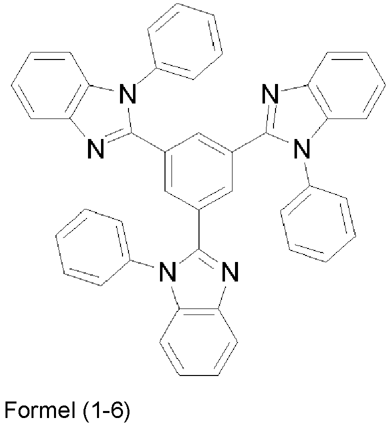 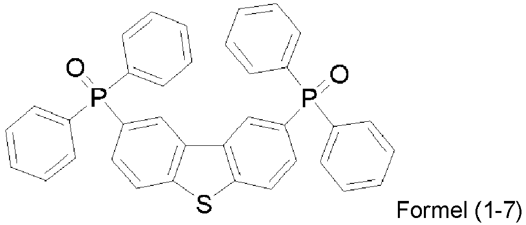 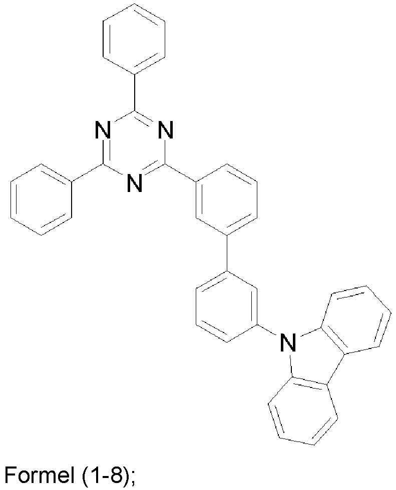 wobei das Material, das die Lochleistungsfähigkeit aufweist, eine oder mehrere Verbindungen der Strukturformeln (2-1) bis (2-7) ist: 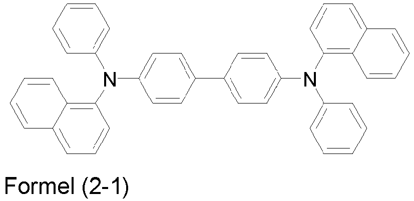 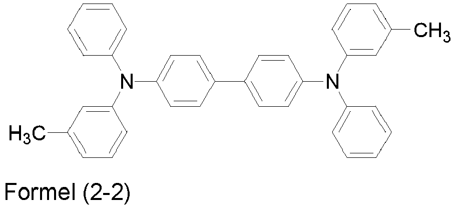 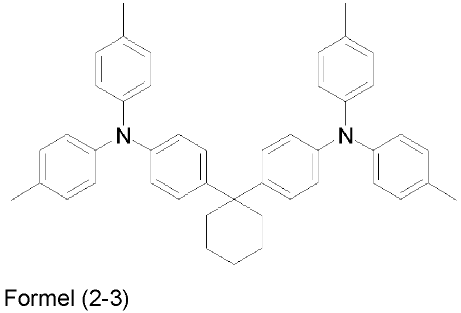 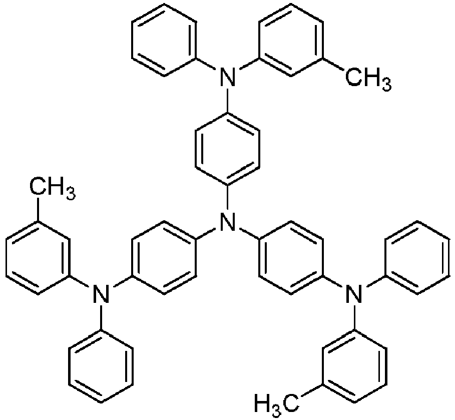 Formel (2-7), und wobei das erste Singulettzustandsenergieniveau des Exciplex höher als das erste Triplettzustandsenergieniveau des phosphoreszierenden Farbstoffs ist.

2. Phosphoreszierende organische Elektrolumineszenzvorrichtung nach Anspruch 1, wobei die Differenz in dem LUMO-Energieniveau zwischen dem Material, das die Lochleistungsfähigkeit aufweist, und dem Material, das die Elektronenleistungsfähigkeit aufweist, wenigstens 0,4 eV ist.

3. Phosphoreszierende organische Elektrolumineszenzvorrichtung nach Anspruch 1, wobei der phosphoreszierende Farbstoff 1-10 Gew.-% der lumineszierenden Schicht ausmacht.

4. Phosphoreszierende organische Elektrolumineszenzvorrichtung nach Anspruch 1, wobei der phosphoreszierende Farbstoff 3 Gew.-% der lumineszierenden Schicht ausmacht.

5. Phosphoreszierende organische Elektrolumineszenzvorrichtung nach Anspruch 1, wobei das Dickeverhältnis der Lochleistungsmaterialschicht zu der Elektronenleistungsmaterialschicht 1:1 bis 1:5 beträgt.

6. Phosphoreszierende organische Elektrolumineszenzvorrichtung nach Anspruch 1, wobei das Dickeverhältnis der Lochleistungsmaterialschicht zu der Elektronenleistungsmaterialschicht 1:3 beträgt.

7. Phosphoreszierende organische Elektrolumineszenzvorrichtung nach Anspruch 1, wobei die phosphoreszierende organische Elektrolumineszenzvorrichtung eine Anode, eine Lochleistungsschicht, die Lochleistungsmaterialschicht, die Elektronenleistungsmaterialschicht, eine Elektronenleistungsschicht und eine Kathode umfasst, die nacheinander auf ein Substrat laminiert sind.

8. Phosphoreszierende organische Elektrolumineszenzvorrichtung nach Anspruch 7, wobei die Anode und die Lochleistungsschicht eine dazwischen angeordnete Lochinjektionsschicht aufweisen.

## Revendications

1. Dispositif électroluminescent organique phosphorescent, comprenant une couche de transport de trous, une couche luminescente et une couche de transport d'électrons, lesquelles sont successivement stratifiées, dans lequel la couche luminescente a une structure à double couche comprenant une couche de matériau de transport de trous et une couche de matériau de transport d'électrons, définissant une interface entre la couche de matériau de transport de trous et la couche de matériau de transport d'électrons, et un exciplexe est formé sur l'interface de la couche de matériau de transport de trous et la couche de matériau de transport d'électrons, l'exciplexe étant un exciplexe TADF présentant un effet fluorescent retardé activé thermiquement ; et la couche de matériau de transport de trous est constituée d'un matériau hôte ayant une capacité de transport de trous, la couche de matériau de transport d'électrons est constituée d'un matériau hôte et d'un colorant phosphorescent dopé dans le matériau hôte, le matériau hôte ayant une capacité de transport d'électrons ; dans lequel la couche de matériau de transport de trous est disposée entre la couche de transport de trous et la couche de matériau de transport d'électrons, la couche de matériau de transport d'électrons étant disposée entre la couche de matériau de transport de trous et la couche de transport d'électrons
dans lequel, le premier niveau d'énergie de l'état triplet du matériau ayant une capacité de transport d'électrons est supérieur au niveau d'énergie de l'état singulet de l'exciplexe, un écart d'énergie entre le premier triplet du matériau ayant une capacité de transport d'électrons et le premier niveau d'énergie de l'état singulet de l'exciplexe étant supérieur ou égal à 0,2 eV ; et la valeur absolue du niveau d'énergie HOMO du matériau ayant une capacité de transport de trous étant inférieure ou égale à 5,3 eV ;
le premier niveau d'énergie de l'état triplet du matériau ayant une capacité de transport d'électrons qui est supérieure au premier niveau d'énergie de l'état singulet de l'exciplexe, un écart d'énergie entre le matériau ayant une capacité de transport d'électrons et l'exciplexe étant supérieur à 0,2 eV ; et la valeur absolue du niveau d'énergie LUMO du matériau ayant une capacité de transport d'électrons étant supérieure à 2,0 eV ; la différence de niveau d'énergie LUMO entre le matériau ayant une capacité de transport de trous et le matériau ayant une capacité de transport d'électrons étant supérieure à 0,3 eV, et la différence de niveau d'énergie HOMO entre le matériau ayant une capacité de transport de trous et le matériau ayant une capacité de transport d'électrons étant supérieure à 0,2 eV ;
dans lequel le matériau ayant une capacité de transport d'électrons est un ou plusieurs des composés des formules structurelles (1-1) à (1-8) : 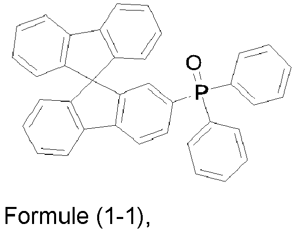 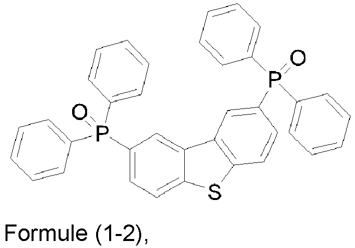 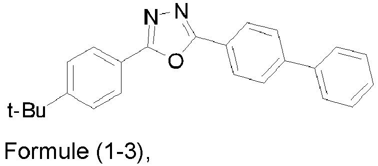 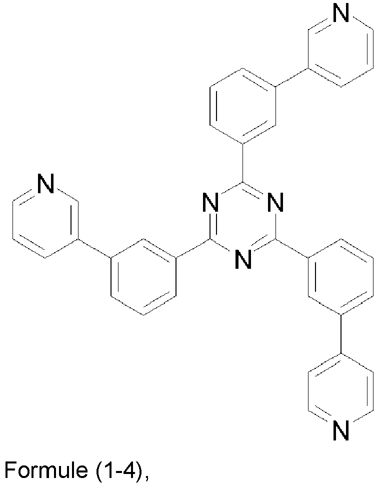 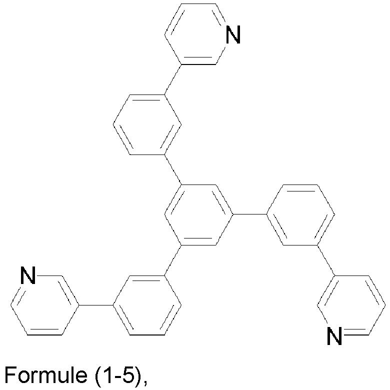 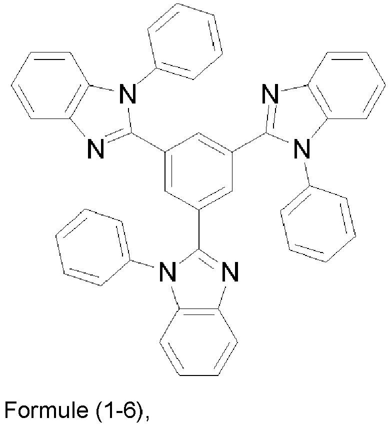 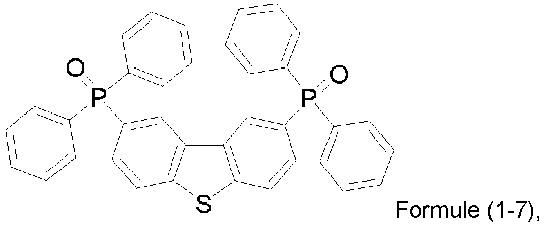 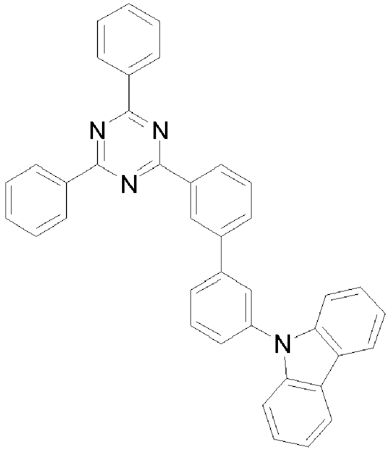 Formule (1-8) ; dans lequel le matériau ayant une capacité de transport de trous est un ou plusieurs des composés des formules structurelles (2-1) à (2-7) : 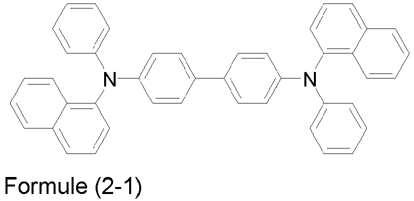 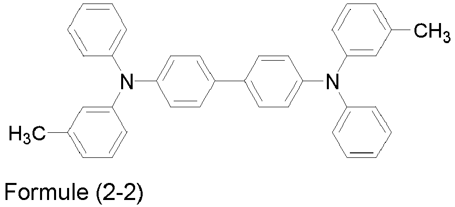 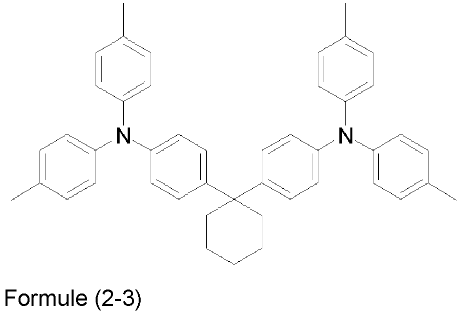 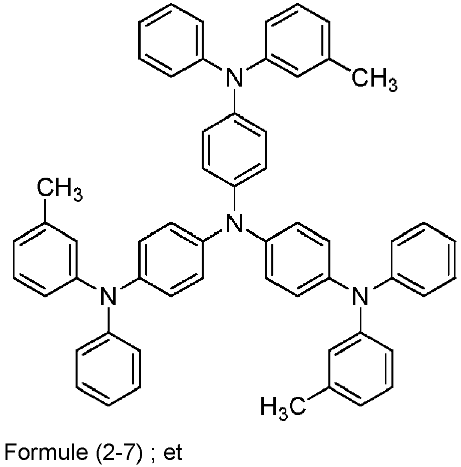 le premier niveau d'énergie de l'état singulet de l'exciplexe est supérieur au premier niveau d'énergie de l'état triplet du colorant phosphorescent.

2. Dispositif électroluminescent organique phosphorescent selon la revendication 1, dans lequel la différence de niveau d'énergie LUMO entre le matériau ayant une capacité de transport de trous et le matériau ayant une capacité de transport d'électrons est supérieure ou égale à 0,4 eV.

3. Dispositif électroluminescent organique phosphorescent selon la revendication 1, dans lequel le colorant phosphorescent représente 1 à 10 % en poids de la couche luminescente.

4. Dispositif électroluminescent organique phosphorescent selon la revendication 1, dans lequel le colorant phosphorescent représente 3 % en poids de la couche luminescente.

5. Dispositif électroluminescent organique phosphorescent selon la revendication 1, dans lequel le rapport d'épaisseur de la couche de matériau de transport de trous à la couche de matériau de transport d'électrons est de 1:1 à 1:5.

6. Dispositif électroluminescent organique phosphorescent selon la revendication 1, dans lequel le rapport d'épaisseur de la couche de matériau de transport de trous à la couche de matériau de transport d'électrons est de 1:3.

7. Dispositif électroluminescent organique phosphorescent selon la revendication 1, le dispositif électroluminescent organique phosphorescent comprenant une anode, une couche de transport de trous, la couche de matériau de transport de trous, la couche de matériau de transport d'électrons, une couche de transport d'électrons et une cathode, lesquelles sont stratifiées successivement sur un substrat.

8. Dispositif électroluminescent organique phosphorescent selon la revendication 7, dans lequel l'anode et la couche de transport de trous ont une couche d'injection de trous disposée entre elles.
